# EUROPÄISCHE PATENTSCHRIFT

(11) **EP 1 056 417 B1**
(45) Veröffentlichungstag und Bekanntmachung des Hinweises auf die Patenterteilung: **20.09.2006**
(21) Anmeldenummer: 99910163.7
(22) Anmeldetag: 29.01.1999
(51) Int. Cl.: A61F 2/18

(54) **MITTELOHRIMPLANTAT**
MIDDLE EAR IMPLANT
IMPLANT POUR OREILLE MOYENNE

(30) Priorität: 18.02.1998 DE 29802776 U
(43) Veröffentlichungstag der Anmeldung: 06.12.2000
(73) Patentinhaber: Spiggle & Theis, 64807 Dieburg (DE)
(72) Erfinder: ELIES, Wolfgang, D-33619 Bielefeld (DE); DALCHOW, Carsten, D-40549 Düsseldorf (DE)
(74) Vertreter: Fuchs Mehler Weiss & Fritzsche
(86) Internationale Anmeldenummer: PCT/EP1999/000574
(87) Internationale Veröffentlichungsnummer: WO 1999/042060

(56) Entgegenhaltungen:
- DE-A- 3 925 670
- DE-U- 29 819 892
- US-A- 3 710 399
- US-A- 4 510 627
- US-A- 4 601 723
- US-A- 4 653 510

## Beschreibung

Die Erfindung bezieht sich auf ein Mittelohrimplantat mit einem flächigen Kopplungskörper zur Anlage an das Trommelfell, einem damit verbundenen länglichen Schaft zur Überbrückung des Raumes in der Paukenhöhle und mit einem mit dem Schaft verbundenen Fuß zur Anlage gegen den Fußpunkt des Implantats in der Paukenhöhle.

Schwerhöhrigkeit bis hin zur Taubheit kann verschiedene pathologische Ursachen haben. Eine der Ursachen liegt typischerweise in einer krankhaften Veränderung bzw. Degeneration der Gehörknöchelchen der Ossikularkette im Mittelohr, d.h. der drei in der Paukenhöhle (Cavum tympani) gelegenen Gehörknöchelchen: Hammer, Amboß und Steigbügel.

Diese Gehörknöchelchen übernehmen die Schalleitung vom Trommelfell über das ovale Fenster (Vorhoffenster) zum Innenohr, wobei die Luft im Mittelohr ein fast reibungsloses Schwingen der gelenkig miteinander verbundenen Gehörknöchelchen ermöglicht. Diese gelenkig miteinander verbundenen Gehörknöchelchen stellen einen Hebelmechanismus dar, der die aufgenommenen Schallwellen beim Menschen um etwa das 2- bis 3-fache verstärkt. Ist der Hebelmechanismus z.B. durch eine degenerative Veränderung der Knochensubstanz gestört, wird der Mensch schwerhörig.

Es ist bekannt, die gestörte Gehörknöchelchenkette operativ total oder teilweise durch eine implantierte Gehörknöchelchen-Prothese, im folgenden als Mittelohrimplantat bezeichnet, zu ersetzen. Diese besteht typischerweise aus einem scheibenförmigen Kopplungskörper zur Anlage gegen das Trommelfell, einem daran angeformten länglichen Schaft zur Überbrückung des freien Raumes in der Paukenhöhle, der durch die operativ entfernten bzw nicht mehr vorhandenen Gehörknöchelchen entsteht, und mit einem Implantatschuh zur breitflächigen Anlage gegen die Fußplatte des Steigbügels bei dem Ersetzen aller drei Knöchelchen (Totalimplantat), sowie einer Implantatglocke zur Anlage an den Steigbügel (Stapes) bei nicht mehr vorhandenem Hammer und Amboß (Partielles Implantat). Das Mittelohrimplantat übernimmt dann ganz ganz oder teilweise die Schwingungsübertragung von dem Trommelfell auf das Vorhoffenster, so daß der Patient wieder das normale Hörvermögen erreicht.

Die einzelnen bekannten Mittelohrimplantate unterscheiden sich im wesentlichen durch die konstruktive Ausgestaltung und durch die Werkstoffwahl.

So zeigt die US-A-4,510,627 eine Gehörknöchelprothese aus einem porösem Kunststoff (Polyethylen) mit einem metallischen Kern hoher Schaltleitfähigkeit. Das poröse Material soll einer einigen Verbindung des Implantates zu dem Trommelfell und der Fußplatte dienen, wogegen der metallische Kern die Funktion der Schallübertragung übernimmt.

Es sind auch Mittelohrimplantate aus Keramik bekannt. Diese haben jedoch den Nachteil, daß sie relativ voluminös sind und mit einem Gewicht von ca. 40 mg eher träge reagieren. Darüber hinaus besteht bei bestimmten Keramiken die Möglichkeit der völligen Zerstörung durch rezidivierende Mittelohrentzündungen. Deshalb sieht die DE 39 01 796 A 1 ein Mittelohrimplantat aus Gold vor, mit dem scheibenförmigen Kopplungs-Körper und dem Fuß aus Feingold und einem Golddraht als Schaft.

Es ist auch aus der DE 42 10 235 C 1 bekannt, bei einem Mittelohrimplantat den flächigen Kopplungskörper zur Anlage an das Trommelfell aus Titan, der mit einer bioaktiven Titanoxidschicht überzogen ist, den Schaft aus Feingold und den Implantatschuh wiederum aus Titan herzustellen.

Bei all diesen Mittelohrimplantaten besteht ein prinzipielles Problem darin, daß die Tiefe der von dem Implantat zu überbrückenden Paukenhöhle bzw. eines Teiles davon von Patient zu Patient unterschiedlich ist, mit der Randbedingung, daß das genaue Maß erst während des operativen Eingriffes ermittelbar ist. Es muß daher sehr schnell ein Implantat mit einem patientenspezifisch längenangepassten Schaft zur Verfügung stehen.

Die erwähnte DE 39 01 796 A 1 sieht zu diesem Zweck die Ausbildung eines Krümmungsbereiches im Schaft vor, der eine Längenreserve bildet, und der es erlaubt, während des operativen Eingriffes die Gesamtlänge des Schaftes den individuellen Gegebenheiten anzupassen. Da das Mittelohrimplantat jedoch extrem klein ist, erfordert die Längenanpassung ein erhebliches Maß an Geschicklichkeit. Auch ist die Stabilität der eingestellten Schaftlänge nicht immer gewährleistet.

Typischerweise werden daher derzeit bei dem operativen Eingriff eine Vielzahl von Mittelohrimplantaten mit unterschiedlich langen Schäften bereitgehalten, von denen dann das den individuellen Gegebenheiten am nächsten kommende Implantat ausgewählt wird. Diese Methode hat den Nachteil eines erheblichen logistischen Aufwandes hinsichtlich der Herstellung, Lagerung und Bereithaltung einer Vielzahl von Mittelohrimplantaten mit unterschiedlich langen Schäften.

Durch die US-A-4 601 723 ist das eingangs bezeichnete Mittelohrimplantat mit einem flächigen Kopplungskörper zur Anlage an das Trommelfell, einem damit verbundenen länglichen Schaft zur Überbrückung des Raumes in der Paukenhöhle und mit einem mit dem Schaft verbundenen Fuß zur Anlage gegen den Fußpunkt des Implantats in der Paukenhöhle, wobei der Fuß und/oder der flächige Kopplungskörper als das zugehörige Ende des Schaftes aufnehmbares Steckteil ausgebildet ist, bekannt geworden. Durch die Ausbildung des flächigen Kopplungskörpers und/oder des Fußes als Steckteile kann zwar erreicht werden, daß nicht komplette Mittelohrimplantate mit unterschiedlich langen Schäften bei der Operation bereitgehalten werden müssen, jedoch besteht weiterhin mit Nachteil das Erfordernis, daß unterschiedlich lange Schäfte auf Vorrat und während der Operation bereitgehalten werden müssen, was einmal ebenfalls die Logistik erhöht und Zeit bei der Auswahl des richtigen Schaftes bei der Operation verbraucht.

Der Erfindung liegt die Aufgabe zugrunde, das eingangs bezeichnete Mittelohrimplantat so auszubilden, daß auf einfache und dennoch wirksame Weise eine Anpassung der Länge des Schaftes des Mittelohrimplantates an die patientenspezifischen Gegebenheiten möglich ist.

Die Lösung dieser Aufgabe gelingt gemäß der Erfindung dadurch, daß der längliche Schaft Soll-Bruchstellen in Form von Durchmesserverjüngungen zur individuellen Anpassung der Länge des Schaftes an die patientenspezifische Tiefe des Baumes der Paukenhöhle während des operativen Eingriffes besitzt, und dadurch, daß der Füß als Steckteil zum Aufsetzen auf das abgelängte Ende des Schaftes ausgebildet ist.

Das erfindungsgemäße Mittelohrimplantat kann daher durch einfaches Abbrechen der überschießenden Länge des Schaftes sehr schnell während des operativen Eingriffes der patientenspezifischen Tiefe des Raumes der Paukenhöhle angepasst werden, wobei anschließend der Fuß oder der flächige Kopplungskörper auf das freie Ende des Schaftes aufgesteckt wird.

Durch das zwar prioritätsjüngere, aber vor dem europäischen Anmeldetag eingetragene deutsche Gebrauchsmuster, veröffentlicht durch das Dokument DE 298 19 892 U1, ist eine Gehörknöchelprothese mit den eingangs bezeichneten Merkmalen bekannt geworden, bei weicher der Fuß einstückig mit einem Ende des Schaftes, der einen konstanten Durchmesser, d.h. keine Soll-Bruchstellen, besitzt, verbunden ist. Das andere Ende des Schaftes ist in einer Öffnung im flächigen Kopplungskörper vercrimpt, wobei vor dem Vercrimpen der Schaft mittels eines Spezialwerkzeuges auf eine patientenspezifische Länge abgelängt werden kann. Weder der Fuß noch der flächige Kopplungskörper sind dabei als Steckteile ausgebildet.

Gemäß einer ersten Weiterbildung kann das Mittelohrimplantat so ausgebildet sein, daß der flächige Kopplungskörper einstückig mit dem Schaft verbunden ist, und der Fuß in Form eines Schuhs zur breitflächigen Anlage gegen die Fußplatte des Steigbügels als Steckteil ausgebildet ist.

In diesem Fall dient das Implantat als Totalprothese, d.h. ersetzt die gesamte Gehörknöchelchenkette.

Der Implantatschuh hat daher bei diesem Mittelohrimplantat gemäß der Erfindung drei Funktionen:
1. Er soll durch seine Basis eine großflächige Auflage an der Fußplatte des Steigbügels (Stapes) gewährleisten, d.h. ein Kippen vermeiden, um so eine stabile Lage des Mittelohrimplantates zu gewährleisten.
2. Durch seine breite Basis, d.h. durch die vergrößerte Fläche, wird ein kleinerer Druck auf die Fußplatte ausgeübt.
3. Er verdeckt wie eine Kappe die Bruchkante und vermeidet daher eine Nacharbeit an der jeweiligen Ist-Trennstelle.

Gemäß einer Weiterbildung der Erfindung sind die Sollbruchstellen in einem vorgegebenen gegenseitigen Abstand zueinander ausgebildet, wobei besondere Vorteile erreicht werden, wenn die gegenseitigen Abstände der Sollbruchstellen unter Vorgabe eines Rastermaßes über die Schaftausdehnung konstant sind.

Eine derartige Ausbildung erlaubt mit Vorteil das Bevorraten von zwei Typen von Implantaten, deren Schaftlänge um einen vorgegebenen Bruchteil des Rastermaßes, vorzugsweise um die Hälfte des Rastermaßes, differieren. Es sind dann mit Vorteil Längenanpassungen um die Hälfte des Rastermaßes möglich.

Vorzugsweise bestehen alle Teile des Mittelohrimplantates aus Titan. Titan ist ein Werkstoff, der leicht, bioverträglich und gut schalleitend ist.

Vorzugsweise sind zumindest der flächige Kopplungskörper und der Schuh mit einer knochenähnlichen Substanz, vorzugsweise Hydroxylapatit, beschichtet. Eine derartige Ausbildung erlaubt mit Vorteil eine sehr innige Verbindung des Implantates mit dem Trommelfell und der Fußplatte, wobei eine geschaffene Rauhigkeit der Oberfläche der Implantatteile förderlich ist.

Gemäß einer anderen Ausgestaltung der Erfindung ist der flächige Kopplungskörper als Scheibe ausgebildet, wobei die Scheibe, symmetrisch verteilt, kreisrunde Öffnungen aufweist. Eine derartige Ausgestaltung ermöglicht mit großem Vorteil eine sehr innige Verwachsung des Kopplungskörpers mit dem Trommelfell.

Gemäß einer Weiterbildung der Erfindung sind der flächige Kopplungskörper mit dem angeformten Schaft einerseits und der Implantatschuh andererseits fertigungstechnisch als Drehteile ausgebildet.

Im Gegensatz zu den Mittelohrimplantaten gemäß dem eingangs zitierten Stand der Technik, die z.T. in mühevoller Handarbeit hergestellt werden müssen, kann das Mittelohrimplantat gemäß der Erfindung auf einem Drehautomat maschinell hergestellt werden.

Weitere Ausgestaltungen und Vorteile der Erfindung ergeben sich anhand der Beschreibung von in den Zeichnungen dargestellten Ausführungsbeispielen der Erfindung.

Es zeigen:
- Fig. 1: in einer Schnittdarstellung eine erste Ausführungsform eines Mittelohrimplantates gemäß der Erfindung mit am Schaft ausgebildeten Sollbruchstellen, deren gegenseitige Abstände unter Vorgabe eines Rastermaßes über die Schaftausdehnung konstant sind, und mit einem einstückig mit dem Schaft verbundenen scheibenförmigen, flächigen Kopplungskörpers zur Anlage an das Trommelfell,
- Fig. 2: ein Mittelohrimplantat entsprechend Fig. 1, bei der jedoch die Schaftlänge um die Hälfte des Rastermaßes größer ist,
- Fig. 3: eine Draufsicht auf den scheibenförmigen flächigen Kopplungskörper nach Fig. 1 oder Fig. 2,
- Fig. 4: in einer teilweise geschnittenen Darstellung den Implantatschuh, der als das freie Ende des Schaftes aufnehmbares Steckteil ausgebildet ist, und
- Fig. 5: eine Draufsicht auf den Fuß nach Fig. 4.

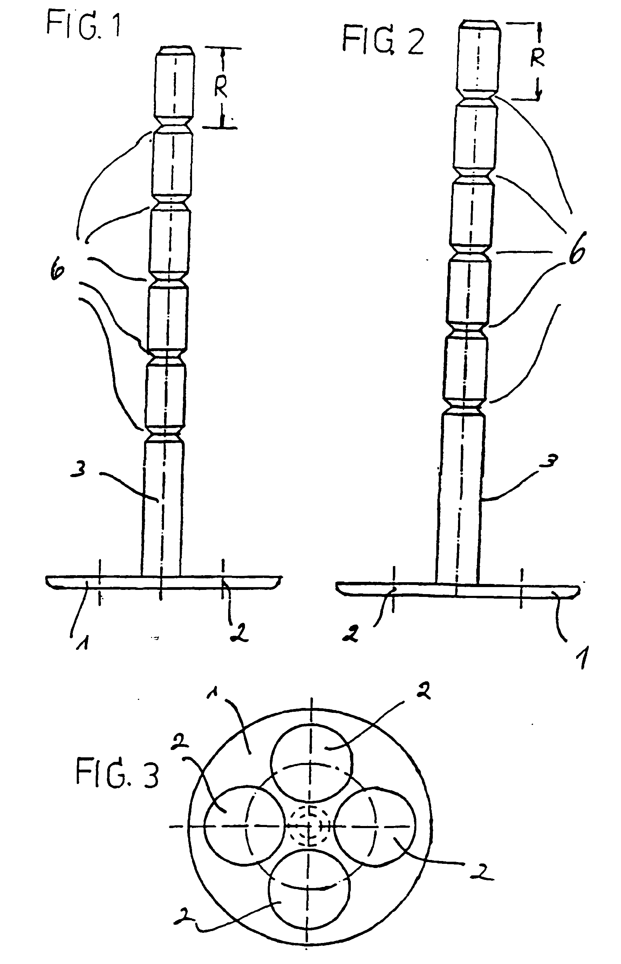

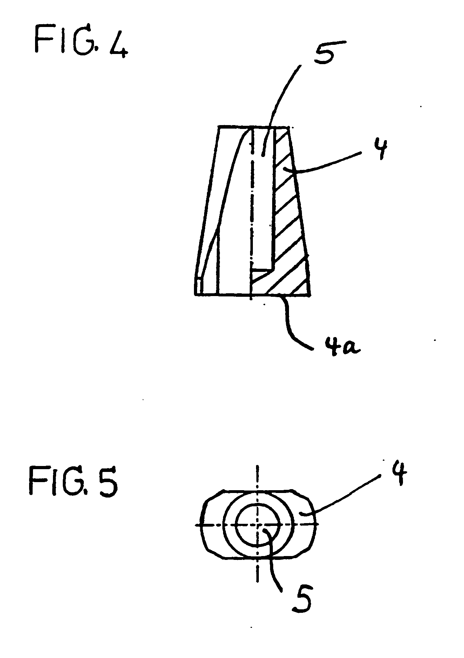

Die Figuren 1 und 4 in Verbindung mit den Draufsichten nach den Figuren 3 und 5 zeigen eine Ausführungsform des erfindungsgemäßen Mittelohrimplantates, die vorzugsweise bei einem Ersatz aller Gehörknöchelchen angewendet wird. Es besitzt einen flächigen Kopplungskörper, der in dem Ausführungsbeispiel als Scheibe 1 ausgebildet ist. Diese Scheibe 1 dient zur Anlage an das Trommelfell eines gehörgeschädigten Patienten und besitzt in dem dargestellten Ausführungsbeispiel vier symmetrisch angeordnete, kreisrunde Bohrungen 2, durch die Trommelfellsubstanz nach der Implantation hindurchwachsen kann, um so eine sichere Verankerung des flächigen Kopplungskörpers, der Scheibe 1 am Trommelfell zu gewährleisten. Mit der koppelnden Scheibe 1 ist ein länglicher Schaft 3 einstückig verbunden, der zur Überbrückung des Raumes in der Paukenhöhle dient. Das freie (obere) Ende des länglichen Schaftes 3 ist durch einen Implantatschuh zur breitflächigen Anlage gegen die Fußplatte des Steigbügels (Stapes) des gehörgeschädigten Patienten abdeckbar. Zu diesem Zweck ist der Schuh 4 gemäß Fig. 4 als Steckteil ausgebildet, mit einer Öffnung 5, in der das freie Ende des Schaftes 3 aufnehmbar ist. Dieser Fuß 4 hat drei Funktionen:
1. Durch die verhältnismäßig große Basisfläche 4 a, die nach der Implantation zur Anlage gegen die Fußplatte des Steigbügels kommt, wird ein Kippen des Mittelohrimplantats verhindert, d.h., er dient der Stabilität des Implantates.
2. Durch die verhältnismäßig große Basisfläche 4 a wird nur ein verhältnismäßig kleiner Druck auf die Fußplatte ausgeübt.
3. Schließlich dient der Schuh 4 zur Verdeckung der Kanten am freien Ende des Schaftes 3, wie noch erläutert werden wird.

Gemäß der Erfindung besitzt der längliche Schaft 3 mehrere Sollbruchstellen 6 in Form von Durchmesserverjüngungen zur individuellen Anpassung der Länge des Schaftes an die patientenspezifische Tiefe des Raumes der Paukenhöhle während des operativen Eingriffes. Zu diesem Zweck wird die individuell überstehende Länge des Schaftes einfach weggebrochen, wobei durch den aufgesteckten Schuh 4 Bruchkanten abgedeckt werden.

Wie in Fig. 1 dargestellt, sind die Sollbruchstellen 6 in einem vorgegebenen gegenseitigen Abstand zueinander ausgebildet, wobei in diesem Ausführungsbeispiel die gegenseitigen Abstände der Sollbruchstellen 6 unter Vorgabe eines Rastermaßes R über die Schaftausdehnung konstant sind. Das Rastermaß R ist abhängig von dem gewählten Material so vorgegeben, daß das überstehende Material ohne Beeinträchtigung der anderen Teile des Schaftes 3 an der jeweiligen Solltrennstelle 6 weggebrochen werden kann.

Um auf sehr einfache Weise noch kleinere patientenspezifische Unterschiede in den Abmessungen der Paukenhöhle gerecht werden zu können, ist gemäß Fig. 2 mit Vorteil ein zweiter Typ eines Mittelohrimplantates vorgesehen, der sich von dem Implantat nach Fig. 1 nur dadurch unterscheidet, daß die Länge des Schaftes 3 um das halbe Rastermaß größer ist. Dadurch lassen sich sehr einfach Schaftlängen, die um ein halbes Rastermaß differieren, verifizieren.

Vorzugsweise bestehen sowohl die Scheibe 1 als auch der daran angeformte Schaft 3 sowie der Fuß 4 aus Titan. Titan ist ein Werkstoff, der leicht, bioverträglich und gut schalleitend ist. Grundsätzlich können jedoch auch andere bioverträgliche Werkstoffe verwendet werden. Vorzugsweise ist sowohl die koppelnde Scheibe 1 als auch der Schuh 4 mit einer knochenähnlichen Substanz, vorzugsweise mit Hydroxylapatit, beschichtet. Auf diese Weise wird eine sehr innige Verbindung des Implantes mit dem Trommelfell einerseits und der Fußplatte andererseits erzielt. Die Oberfläche der Scheibe und des Fußes sind vorzugsweise aufgerauht.

Bei der Ausführungsform nach den Figuren 1 und 2 ist der flächige Kopplungskörper als Scheibe 1 ausgebildet. Auch hierbei sind andere Ausführungsformen denkbar, z.B. die Ausbildung des Kopplungskörpers als Speichenrad.

Wie die Figuren erkennen lassen, sind sowohl der Kopplungskörper mit dem angeformten Schaft 3 einerseits und der Implantatschuh 4 andererseits fertigungstechnisch als Drehteile ausgebildet, so daß das erfindungsgemäße Mittelohrimplantat durch einen Drehautomaten maschinell herstellbar ist, im Gegensatz zu den bekannten Implantaten, die z.T. eine mühevolle Handarbeit erfordern.

Das in den Figuren 1-5 dargestellte Mittelohrimplantat besitzt typischerweise nachstehende Abmessungen:
- Durchmesser der Scheibe 1: 3 mm bei einer Scheibendicke von 0,15 mm.
- Der Durchmesser des Schaftes 3 beträgt ca. 0,4 mm.
- Das Rastermaß R beträgt ca. 1 mm.
- Die Länge des Schaftes 3 beträgt bei der Ausführung nach Fig. 1 7 mm, bei der Ausführung nach Fig. 2 jedoch 7,5 mm.
- Der Durchmesser der Bohrungen 2 beträgt 1 mm.
- Die Höhe des Schuhes 4 beträgt 2 mm mit einer Sackbohrung 5 von 1,7 mm Länge.
- Die Basisfläche 4 a des Schuhes beträgt 1,3 mm bei einer Schuhbreite von 0,8 mm.

Wenn der Schaftdurchmesser kleiner als 0,4 mm, z.B. 0,2 oder 0,3 mm, beträgt, dann sind die die Sollbruchstellen 6 bildenden Ausnehmungen im Schaft 3 weniger ausgeprägt. Die Sollbruchstellen haben generell neben der Funktion, daß der Schaft an diesen Stellen leichter brechen soll, bzw. dort eine Trennung zu erfolgen hat, noch die Funktion, daß an dieser Stelle ein geringerer Schaftdurchmesser gegeben ist, der das Aufstecken des Implantat-Schuhes erleichtern soll.

Mitunter ist es bei der Implantierung notwendig, den scheibenförmigen Kopplungskörper 1 gegenüber dem Schaft 3 zu neigen, um der vorgegebenen Anatomie des Patientenohres Rechnung zu tragen. Zur Erleichterung dieses Abbiegens ist zweckmäßig der Schaft 3 im Bereich des Scheibenansatzes schmaler als im übrigen Bereich ausgebildet.

## Patentansprüche

1. Mittelohrimplantat mit einem flächigen Kopplungskörper (1) zur Anlage an das Trommelfell, einem damit verbundenen länglichen Schaft (3) zur Überbrückung des Raumes in der Paukenhöhle und mit einem mit dem Schaft (3) verbundenen Fuß (4) zur Anlage gegen den Fußpunkt des Implantats In der Paukenhöhle, **dadurch gekennzeichnet, daß** der längliche Schaft (3) Soll-Bruchstellen (6) in Form von Durchmessorverjüngungen zur individuellen Anpassung der Länge des Schaftes an die patientenspezifische Tiefe des Raumes der Paukenhöhle während des operativen Eingriffes besitzt, und daß der Fuß als Steckteil zum Aufsetzen auf das abgelängte Ende des Schaftes (3) ausgebildet ist.

2. Mittelohrimplantat nach Anspruch 1, **dadurch gekennzeichnet, daß** der flächige Kopplungskörper (1) einstückig mit dem Schaft verbunden ist, und der Fuß in Form eines Schuhs (4) zur breitflächigen Anlage gegen die Fußplatte des Steigbügels als Steckteil ausgebildet ist.

3. Mittelohrimplantat nach Anspruch 1 oder 2, **dadurch gekennzeichnet, daß** die Solltrennstellen (6) in einem bestimmten gegenseitigen Abstand zueinander ausgebildet sind.

4. Mittelohrimplantat nach Anspruch 3, **dadurch gekennzeichnet, daß** die gegenseitigen Abstände der Solltrennstellen, unter Definition eines Rastermaßes (R), über die Schaftausdehnung konstant sind.

5. Mittelohrimplantat nach Anspruch 4, **dadurch gekennzeichnet, daß** zwei Typen von Prothesen vorgesehen sind, deren Schaftlänge um einen bestimmten vorgegebenen Bruchteil des Rastermaßes differiert.

6. Mittelohrimplantat nach Anspruch 5, **dadurch gekennzeichnet, daß** die Differenz die Hälfte des Rastermaßes (R) beträgt.

7. Mittelohrimplantat nach einem der Ansprüche 1 bis 6, **dadurch gekennzeichnet, daß** der flächige Kopplungskörper als Scheibe (1) ausgebildet ist.

8. Mittelohrimplantat nach Anspruch 7, **dadurch gekennzeichnet, daß** die Scheibe (1), symmetrisch verteilt, kreisrunde Öffnungen (2) aufweist.

9. Mittelohrimplantat nach einem der Ansprüche 1 bis 8, **dadurch gekennzeichnet, daß** der flächige Kopplungskörper (1) mit dem angeformten Schaft (3) einerseits und der Fuß (4) andererseits fertigungstechnisch als Drehteile ausgebildet sind.

## Claims

1. Middle ear implant with a planar coupling body (1), for application to the eardrum, with an elongate shaft (3) connected to the coupling body (1) and used for bridging the space in the tympanic cavity and with a base (4) which is connected to the shaft (3) and which bears against the base point of the implant in the tympanic cavity, **characterized in that** the elongate shaft (3) has predetermined break points (6) in the form of diameter constrictions for individually adapting the length of the shaft to the patient-specific depth of the space of the tympanic cavity during the surgical intervention, and **in that** the base (4) is formed as a plug part for fitting onto the sectioned end of the shaft (3).

2. Middle ear implant according to Claim 1, **characterized in that** the planar coupling body (1) is connected integrally to the shaft, and **in that** the base is designed as a plug part in the form of a shoe (4) for bearing against a wide surface area of the foot-plate of the stirrup.

3. Middle ear implant according to Claim 1 or 2, **characterized in that** the predetermined separation points (6) are formed at a defined mutual spacing from one another.

4. Middle ear implant according to Claim 3, **characterized in that** the mutual spacings of the predetermined separation points are constant along the extent of the shaft, thereby defining a modular dimension (R).

5. Middle ear implant according to Claim 4, **characterized in that** two types of prostheses are provided whose shaft length differs by a given predetermined fraction of the modular dimension.

6. Middle ear implant according to Claim 5, **characterized in that** the difference amounts to half of the modular dimension (R).

7. Middle ear implant according to one of Claims 1 to 6, **characterized in that** the planar coupling body is designed as a disc (1).

8. Middle ear implant according to Claim 7, **characterized in that** the disc (1) comprises circular openings (2) distributed in a symmetrical arrangement.

9. Middle ear implant according to one of Claims 1 to 8, **characterized in that** the planar coupling body (1) with integrally formed shaft (3) on the one hand, and the base (4) on the other hand, are machined as turned parts.

## Revendications

1. Implant pour l'oreille interne, qui présente un corps plat d'accouplement (1) en vue de son installation sur le tympan, une tige allongée (3) qui est reliée au corps d'accouplement de manière à traverser l'espace de la cavité du tympan et un pied (4) relié à la tige (3) et destiné à être installé contre la base de l'implant dans la cavité tu tympan, **caractérisé en ce que** la tige allongée (3) présente des emplacements de rupture (6) qui ont la forme de rétrécissements du diamètre pour adapter la longueur de la tige à la profondeur de l'espace de la cavité du tympan, propre à chaque patient individuel, pendant l'opération et **en ce que** le pied (4) est configuré en pièce enfichable destiné à être monté sur l'extrémité raccourcie de la tige (3).

2. Implant pour l'oreille moyenne selon la revendication 1, **caractérisé en ce que** le corps plat d'accouplement (1) est relié d'un seul tenant à la tige et **en ce que** le pied est configuré en pièce enfichable en forme de soulier (4) destiné à être placé sur une grande surface de la plaque de base de l'étrier.

3. Implant pour l'oreille moyenne selon les revendications 1 ou 2, **caractérisé en ce que** les emplacements de rupture (6) présentent entre eux une distance déterminée.

4. Implant pour l'oreille moyenne selon la revendication 3, **caractérisé en ce que** la distance entre deux emplacements de rupture est constante sur toute la longueur de la tige et définit une dimension modulaire (R).

5. Implant pour l'oreille moyenne selon la revendication 4, **caractérisé en ce qu'**il prévoit deux types de prothèses dont la longueur de tige diffère d'une fraction prédéterminée de la dimension modulaire.

6. Implant pour l'oreille moyenne selon la revendication 5, **caractérisé en ce que** la différence est égale à la moitié de la dimension modulaire (R).

7. Implant pour l'oreille moyenne selon l'une des revendications 1 à 6, **caractérisé en ce que** le corps plat d'accouplement est configuré en disque (1).

8. Implant pour l'oreille moyenne selon la revendication 7, **caractérisé en ce que** le disque (1) présente des ouvertures (2) circulaires et réparties symétriquement.

9. Implant pour l'oreille moyenne selon l'une des revendications 1 à 8, **caractérisé en ce que** le corps plat d'accouplement (1) et la tige (3) qui y est formée, d'une part, et la base (4), d'autre part, sont fabriqués au tour.
